(19) Europäisches Patentamt
European Patent Office
Office européen des brevets

(11) **EP 2 278 026 A1**

(12) **EUROPEAN PATENT APPLICATION**

(43) Date of publication:
**26.01.2011 Bulletin 2011/04**

(51) Int Cl.:
***C12Q 1/68*** (2006.01)

(21) Application number: **09305701.6**

(22) Date of filing: **23.07.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicants:
• **Université de la Méditerranée**
**13284 Marseille Cédex 07 (FR)**

• **Assistance Publique Hôpitaux de Marseille**
**13354 Marseille Cedex 5 (FR)**

(72) Inventor: **Taranger-Charpin, Colette**
**13260 Cassis (FR)**

(74) Representative: **Chajmowicz, Marion et al**
**Becker & Associés**
**25, Rue Louis le Grand**
**75002 Paris (FR)**

(54) **A method for predicting clinical outcome of patients with breast carcinoma**

(57) The invention provides an *in vitro* method for predicting clinical outcome of a patient affected with breast carcinoma, which method comprises determining the expression of proteins including P13K, Moesin, pA-KT, and/or pMAP-K in a biological sample of said patient.

EP 2 278 026 A1

## Description

The invention provides methods for classifying patients affected with breast carcinoma, and identifying node-negative cases who do not need chemotherapeutic treatment.

## Technical background:

The incidence of early-stage breast carcinomas, particularly lymph-node-negative cases, has increased with the implementation of breast cancer screening programs in developed countries. Patients with node-negative breast cancer have fairly good ten-year overall survival with loco-regional treatment alone, only 30-40 % developing distant metastases (1). However, most patients are offered chemotherapy according to current guidelines, leading to over-treatment of a large proportion (2, 3), since there is no means of clearly identifying those patients who will not relapse and hence do not need adjuvant chemotherapy. Therefore, markers to identify patients not requiring aggressive adjuvant therapy are urgently needed, so as to avoid unnecessary exposure of women to the potential toxicity and side-effects of such treatment, and also to reduce the overall cost of breast cancer management.

In this respect, the recent development of new high-throughput methods such as gene expression microarray techniques gives great promise for the identification of prognostic classifiers (4-11). Several interesting studies have reported gene signatures predictive of prognosis in node-negative breast carcinomas (10, 12, 13).

However, these assays (i) usually require frozen tissue, that is not always easy to obtain or handle in routine clinical practice (or tissue briefly fixed in specific fixatives that are most often not appropriate for microscopic diagnosis), (ii) require a large amount of tissue although in small tumors detected at an early stage, the sampling can be detrimental to pathological diagnosis, and (iii) have very high cost, that is unlikely to be supportable by either an individual patient or public health insurance. Also, it has been shown that the large amount of data provided by gene expression microarrays is somewhat liable to misinterpretation (14-16).

In light of the above, there is still a need for predicting clinical outcome of patients with breast carcinoma, and identifying those who would benefit from a chemotherapeutic treatment, vs. those for whom a chemotherapeutic treatment is not recommended, or might even be detrimental.

## Summary of the invention

The present invention provides a molecular signature for predicting clinical outcome in a patient affected with early stage breast carcinoma.

More particularly the invention provides an in vitro method for predicting clinical outcome of a patient affected with breast carcinoma, which method comprises determining the expression level of proteins.

The invention provides an *in vitro* method for predicting clinical outcome of a patient affected with a breast carcinoma, which method comprises determining the expression of at least two proteins in a biological sample of said patient, wherein on of them is PI3K and one other is Moesin, pAKT, or pMAP-K (phospho MAPKAPK-2).

Preferably-the patient is affected with an node-negative breast carcinoma.

Advantageously, expression of said proteins is indicative of a patient with poor clinical outcome or who would benefit from a chemotherapeutic treatment.

The combined expression profile of these proteins is informative of the status of the patient who, before any chemotherapeutic treatment, can be classified as (i) at very early stage of the disease and for whom a chemotherapeutic treatment is not recommended, or might even be detrimental, vs (ii) at advanced stage, i.e. exhibiting a poor clinical outcome and who would benefit from a chemotherapeutic treatment.

In a particular embodiment, the method of the invention comprises quantifying the expression level of said proteins and subjecting the obtained values to a logistic regression, such as a multivariate fractional polynomial logistic regression, whereby a risk score is obtained which correlates to the probability of the patient to show a poor clinical outcome.

More particularly, the probability of poor outcome may be calculated as:

$$\text{Probability of poor outcome} = \exp(-\text{risk score}) / [1 + \exp(-\text{risk score})]$$

## FIGURE LEGENDS

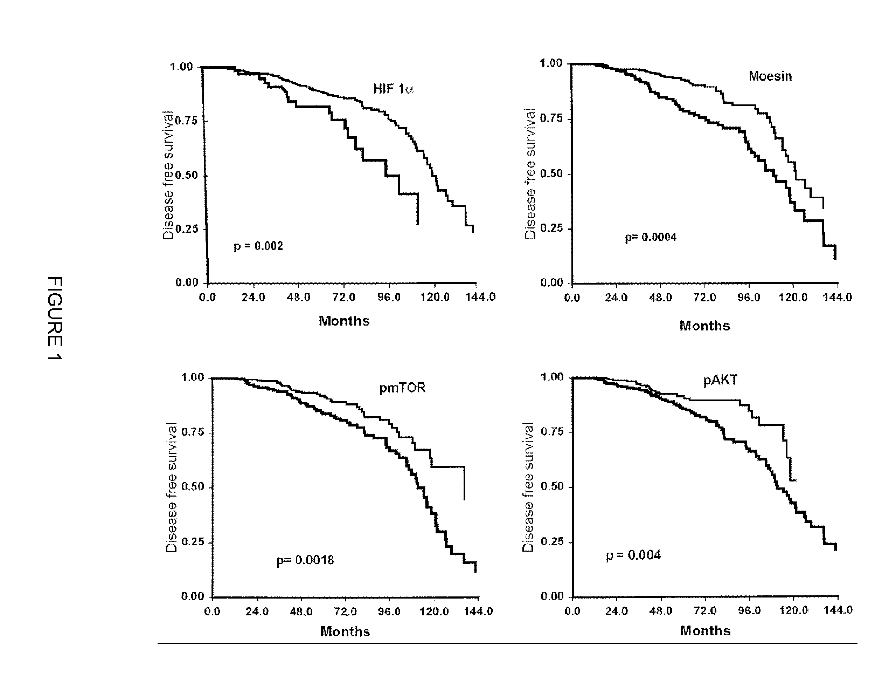

**Figure 1** - Kaplan-Meier curves (log rank test) determining prognostic significance of markers (as shown for HIF-1α, Moesin, pmTOR and pAKT) quantitative scores in 572 node-negative breast carcinomas (TMA, quantitative immunochemical assays).

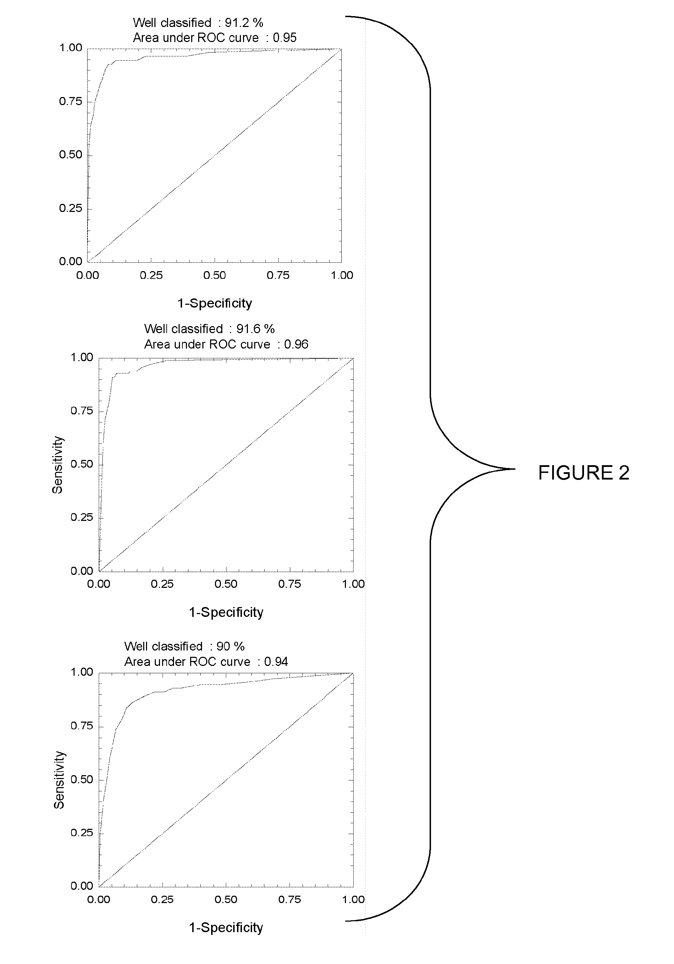

**Figure 2** - First and second steps of logistic regression and ROC curves determining the signature (PI3K pmTOR, pMAP-K, SHARP-2, P21, HIF-1α, Moesin, p4EBP-1, pAKT and P27) that correctly classified node-negative patients (91.4 % and 90 % top and bottom respectively in the category of favorable and unfavorable prognosis). A second regression assessed independently of ER, PR and c-erb B2 status also correctly classified 91.6 % of the patients (middle). Quantitative immunohistochemical assays on TMA (n = 572 node-negative breast carcinomas).

## Detailed description of the invention:

The inventors have now identified an immunohistochemical signature of poor prognosis, and particularly of risk of early distant metastasis, in cases of node-negative breast carcinoma, that is economically acceptable for individual patients with breast carcinoma, using standardized methods. More particularly, the inventors assessed 64 immunocytochemical markers (a large panel of known prognostic markers of tumor cell growth and proliferation, invasion and scattering, and angiogenesis, in addition to markers of signalling pathways) by quantitative immunohistochemistry using a SAMBA / TRIBVN device (22-28) that provides accurate numerical data for statistical analysis (log rank, logistic regression and clustering) of continuous variables, with high-throughput standardized assays using tissue microarrays (TMAs) (29-41) in a large retrospective series (n = 667) of node-negative breast carcinomas.

In the present study, the inventors correlated the quantified immunohistochemical expression of each marker, then of groups of markers (referred to as immunohistochemical signatures), with patient's outcome (mean follow-up 102 months, SD = 14), in order to identify the combination of markers with the best sensitivity and specificity to predict prognosis in terms of occurrence of early distant metastases, using a multivariable fractional polynomials method. This approach allowed to use a continuous variable (densitometry of markers in immunoexpression) so that the correlation between a continuous predictor and the outcome variable could be evaluated in some form of nonlinearity (46, 47).

*Patients*

The term "patient" refers to any subject , preferably a human female afflicted with a breast carcinoma.

Preferably the patient is affected with a node-negative breast carcinoma, that is to say that no metastasis has been detected.

In practice, the determination of the expression of proteins, e.g. by immunocytochemistry, offers a powerful tool for classifying patients and identifying those who are of worst prognostic and would benefit from a chemotherapeutic treatment, avoiding unnecessary cost effective treatment.

*Clinical outcome*

In the context of the present invention, the term "clinical outcome" refers to the risk of metastasis, in particular remote or distant metastasis in the tested patient.

The molecular signatures of the invention are indicative of early distant metastasis. More particularly, the present invention allows thus it to identify "high risk" patients who would benefit from a chemotherapeutic treatment. By extension, node-negative patients designated 'low risk' by the integrated signature might consider not undergoing chemotherapy treatment. The chemotherapy that is herein contemplated is more preferably an adjuvant chemotherapy, i.e. a chemotherapy treatment combined with or set after a surgical intervention.

*The sets of predictive proteins*

All the proteins identified are known per se, and listed in the below tables (Table 1A, and Table 1B).

Table 1A: Proteins of the preferred signatures

| Name of the protein | Genbank Access Number (Examples of human proteins) |
|---|---|
| PI3K (Phosphatidyl Inositol 3 Kinase) | CAA03853 |
| Moesin | NP_002435 |
| P21 (cyclin kinase inhibitor) | AAB29246 |
| phosphorylated AKT (pAKT) | NP_001014432 |
| SHARP2 (basic helix-loop-helix family, member e40) | NP_003661 |
| pMAP-K (also named phosphorylated or phospho MAPKAPK-2) (MAP kinase) | NP_116584 |
| PmTOR (phosphorylated) (mammalian Target Of Rapamycin) | NP_004949 |
| p4E-BP-1 | NP_004086 |
| P27 (protein P) | BAA25263 |
| HIF-1$\alpha$ (Hypoxia-Inducible Factor 1, alpha subunit) | NP_001521 |
| PTEN (Phosphatase and Tensin homolog) | AAD13528 |
| GATA 3 (GATA binding protein 3) | CAH73143 |
| VEGF (vascular endothelial growth factor) | CAC19516 |
| SHARP 2 | NP_003661 |
| STAT-1 (signal transducer and activator of transcription 1) | NP_009330 |
| Caveolin (CAV1) | NP_001744 |

The markers included in the signature include mainly molecules involved in the principal signalling pathways within abnormal tumor cells with altered cell machinery. Most of these markers have been individually reported as dysfunctional in tumors (see reviews in 28, 42, 43) and described as potential targets for specific therapy. Interestingly, some are involved in the mTOR pathway (PI3K, AKT, 4EBP-1) and other signalling networks. The mTOR (mammalian Target Of Rapamycin) protein shows aberrant high activity in breast cancers, as well as other gynecological malignancies, that induces increased tumor cell metabolism and growth, and has been proposed as an interesting target for specific therapy. Phase I and II trials have shown that molecules such as the rapamycin analogues temsirolimus (CCI 799), everolimus (RAD CO1) and ARIAD (AP 23573), that specifically inhibit the mTOR protein, have significant antitumor activity against gynecological cancers (48). Likewise, in breast cancers, molecules blocking mTOR activity increase sensitivity to anthracyclines and taxans (49) and show synergistic action with letrozole (50).

Overexpression of moesin in breast carcinomas has recently been reported in basal-like carcinomas (35). It is therefore noteworthy that moesin may also be overexpressed and indicative of prognosis, in association with the other markers of the signature that the inventors have identified in node-negative tumors that are associated with an unfavorable patient outcome, and so require more aggressive treatment.

HIF-1$\alpha$ and downstream molecules including PI3K, AKT and MAPK constitute a major transcriptional factor in nutrient stress signalling, and the role of HIF-1$\alpha$ is crucial for development of anticancer therapy (51). In a previous study (52), it was shown that immunoexpression of HIF-1$\alpha$ in large sections of breast carcinoma was prognostically predictive in univariate analysis in node-negative tumors. In malignancies, translation of HIF-1$\alpha$ has been found to up-regulate mTOR through upstream mTOR activators including PI3K and AKT (53), suggesting that the effects of HIF-1$\alpha$ on tumor

cell metabolism and growth can be blocked through mTOR inactivation by PI3K and AKT inhibitors (54).

HIF-1α also mediates VEGF angiogenic machinery, acting on tumor neoangiogenesis through PI3K, AKT, phospho MAPKAPK-2 and other factors including the Ras-erk pathways (51, 55). Blockage of VEGF by monoclonal antibodies or by anti-VEGFR molecules such as sunitinib, sorafenib or vatalanib may act to reduce tumor growth due to overexpression of HIF-1α in tumor cells (see reviews in 51, 52, 55).

More particularly, the method of the invention comprises determining the expression of the proteins selected from the groups consisting of:

a. PI3K and Moesin (which correctly classifies 71% of cases in the group of patients with metastasis, using logistic regression)

b. PI3K and pAKT and phospho MAPKAPK-2 (which correctly classifies 78% of cases in the group of patients with metastasis, using logistic regression) and

c. PI3K and Moesin and phospho MAPKAPK-2 (which correctly classifies 83% of cases in the group of patients with metastasis, using logistic regression)

The method may further comprise determining the expression of P21.

Determination of PI3K, Moesin, P21 and pAKT correctly classifies 86% of cases in the group of patients with metastasis, using logistic regression.

The method may further comprise determining the expression of SHARP2 and/or pMAP-K phospho MAPKAPK-2.

The method may further comprise determining the expression of pmTOR and/or VEGF.

In an embodiment, the method of the invention comprises determining the expression of PI3K, Moesin, P21, pAKT, SHARP2, pMAP-K, and optionally HIF1α.

In another embodiment, the method of the invention comprises determining the expression of PI3K, Moesin, P21, pAKT, pMAP-K, VEGF, and optionally pmTOR.

Determination of PI3K, Moesin, P21, pAKT, SHARP2 and pMAP-K correctly classifies 88% of cases in the group of patients with metastasis, using logistic regression.

Determination of PI3K, Moesin, P21, pAKT, SHARP2, pMAP-K and HIF-1α correctly classifies 86% of cases in the group of patients with metastasis, using logistic regression.

In a preferred embodiment, the method of the invention comprises determining the expression of PI3K, Moesin, P21, pAKT, SHARP2, pMAP-K, pmTOR, p4E-BP-1, P27, and HIF-1α. This method correctly classifies 90.4% of cases in the group of patients with metastasis, using logistic regression

Alternatively, in a preferred embodiment, the method of the invention comprises determining the expression of PI3K, Moesin, P21, pAKT, pMAP-K, pmTOR, p4E-BP-1, P27, PTEN, Gata3 and VEGF. This method correctly classifies 90.6% of cases in the group of patients with metastasis, using logistic regression.

Determination of PI3K, Moesin, P21, pAKT, pMAP-K, VEGF and pmTOR correctly classifies 89.2% of cases in the group of patients with metastasis, using logistic regression.

Determination of PI3K, Moesin, P21, pAKT, pMAP-K and VEGF correctly classifies 86% of cases in the group of patients with metastasis, using logistic regression. In still a further embodiment, the method of the invention comprises determining the expression of PI3K, STAT-1, P21, VEGF, pMAP-K, moesin, pAKT, caveolin, p4EBP-1, and pmTOR. This signature has shown a valuable predictive performance in patients with breast carcinomas, independently of their status with respect to metastasis of nodes. Indeed, this method correctly classifies 88.84% of cases in the group of patients, using logistic regression.

In a particular aspect of the invention, the method comprises determining the expression of at least 30 genes, preferably at least 40 genes, preferably at least 50 genes, selected from the group of genes as shown in Table 1 B. In a particular embodiment, the method comprises determining the expression of the 64 markers of Table 1 B.

Table 1B. List of 64 markers, and antibodies against them

|  | | ANTIBODIES | SUPPLIER | SOURCE* | CLONE |
|---|---|---|---|---|---|
| | 1 | MMP7 | Abeam | Rpab | |
| | 2 | MMP11 | Abeam | Rmab | EP1259Y |
| | 3 | eIF4E | Cell signaling | Rmab | C46H6 |
| | 4 | p70 S6 Kinase | Cell signaling | Rmab | 49D7 |
| | 5 | FoxO3a | Cell signaling | Rpab | |

(continued)

|  | ANTIBODIES | SUPPLIER | SOURCE* | CLONE |
|---|---|---|---|---|
| 6 | p42 MAP Kinase erk2 | Cell signaling | Rpab | |
| 7 | AF6 | BD Biosciences | Rmab | 35 |
| 8 | YB1 | Abeam | Mpab | |
| 9 | Phospho-mTOR (Ser2448) | Cell signaling | Rmab | 49F9 |
| 10 | PTEN2 | Signet COVANCE | Mmab | 6H2.1 |
| 11 | VEGF | R&D Systems | Mmab | 26503 |
| 12 | Phospho-4E-BPI(Thr37/46) | Cell signaling | Rmab | 236B4 |
| 13 | HIF 1 alpha | Sift Pouyssegur | 50 | 729T3 |
| 14 | MDR | Abeam | Mmab | JSB1 |
| 15 | Topoisomerase II α | Dako | Mmab | Ki-S1 |
| 16 | Beta-Catenin | Novocastra | Mmab | 17C2 |
| 17 | GATA-3 | Santa Cruz | Mmab | HG3-31 |
| 18 | FGFR-1 Flg (C-15) | Santa Cruz | Rpab | |
| 19 | Maspin | BD Pharmingen | Mmab | G167-70 |
| 20 | C-Met-2 | Chemicon/Abcys | Mmab | 4AT44 |
| 21 | P-Cadherin | Novocastra | Mmab | 56C1 |
| 22 | Ezrin (p81, 80k, cytovillin) | Neomarkers | Mmab | 3C12 |
| 23 | Phospho-AKT (Ser473) | Cell Signaling | Rmab | 587F11 |
| 24 | CD 44v6 | Novocastra | Mmab | VFF-7 |
| 25 | CD44 (HCAM) | Novocastra | Mmab | F10-44-2 |
| 26 | MoesinBio | Biomeda | Mmab | 38/87 |
| 27 | MoesinNeo | Neomarkers | Mmab | 38/87 |
| 28 | Cytokeratins 8 & 18 | Zymed | Mmab | Zym5,2(UCD/PR-10,1 |
| 29 | Cytokeratin 17 | Dako | Mmab | E3 |
| 30 | Cytokeratin 14 | Novocastra | Mmab | LL002 |
| 31 | Phospho-Stat3(Tyr705) | Cell Signaling | Rmab | D3A7 |
| 32 | Melan A | Dako | Rmab | A103 |
| 33 | CD 10 | Novocastra | Mmab | 56C6 |
| 34 | CD 34 | Dako | Mmab | QBEnd-10 |
| 35 | Vimentine | Immunotech | Mmab | V9 |
| 36 | Cytokeratin 19 | Dako | Mmab | BA17 |
| 37 | Phospho-MAPKAPK-2 | Cell Signaling | Rmab | (Thr334) |
| 38 | EGFR | Ventana | Mmab | 3C6 |
| 39 | STAT-1 | Cell Signaling | Mmab | 9H2 |
| 40 | FAK | Cell Signaling | Rpab | |
| 41 | P38 MAP kinase | Cell Signaling | Rpab | |
| 42 | P27 Kip1 | Cell Signaling | Rpab | |
| 43 | P21Waf1-Cipl | Cell Signaling | Mmab | DCS60 |

(continued)

| | | ANTIBODIES | SUPPLIER | SOURCE* | CLONE |
|---|---|---|---|---|---|
| | 44 | SHARP 2 | Abeam | Rpab | |
| | 45 | FYN | Abeam | Mmab | 1S |
| | 46 | p63 | Dako | Mmab | 4A4 |
| | 47 | Cytokeratine 903 | Dako | Mmab | 34BE12 |
| | 48 | CA IX | Abeam | Rpab | |
| | 49 | E-Cadherine | Zymed | Mmab | 4A2C7 |
| | 50 | CD 117 (c-Kit) | Dako | Rpab | |
| | 51 | Cytokeratins 5-6 | Dako | Mmab | D5/16B4 |
| | 52 | PTEN | Cell Signaling | Mmab | 26H9 |
| | 53 | PI3 kinase | Cell Signaling | Rpab | |
| | 54 | JAK 1 | Cell Signaling | Rpab | |
| | 55 | c-Met | Novocastra | Mmab | 8F11 |
| | 56 | Caveolin 1 | Santa Cruz | Rpab | |
| | 57 | CD 105 | Dako | Mmab | SN6h |
| | 58 | CD 146 | Novocastra | Mmab | N1238 |
| | 59 | Bc12 | Dako | Mmab | 124 |
| | 60 | P53 | Dako | Mmab | DO-7 |
| | 61 | P16 | Neomarkers | Mmab | Ab7(16P07) |
| | 62 | c-erbB2 | Novocastra | Mmab | CB11 |
| | 63 | **Progesteron receptor (PR)** | Ventana | Mmab | 1E2 |
| | 64 | **Estrogen receptor (ER)** | Ventana | Mmab | 6F11 |

*Determination of expression*

Determination of the expression of a protein or gene can be performed by a variety of techniques, from a biological sample.

The term "determination of the expression" means detecting the presence of the protein and/or quantifying the level of expression of the protein. The term "biological sample" means any biological sample derived from a patient. Examples of such samples include fluids, tissues, cell samples, organs, biopsies, etc. Most preferred samples are breast tumor samples, most preferably a tumor tissue biopsy. Blood, plasma, saliva, urine, seminal fluid, etc, may also be used.

Preferably, the determination comprises contacting the sample with selective reagents such as probes, primers or ligands, and thereby detecting the presence, or measuring the amount, of polypeptide or nucleic acids of interest originally in the sample. Contacting may be performed in any suitable device, such as a plate, microtiter dish, test tube, well, glass, column, and so forth In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the sample.

A preferred method for determining the expression of said proteins includes detecting and/or quantifying proteins by immunological reaction.

Such methods comprise contacting a biological sample with a binding partner capable of selectively interacting with a marker protein present in the sample. The binding partner is generally an antibody, that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin immunoperoxidase type assays; radioimmunoassays; immunoelectrophoresis; immunoprecipition, etc. Also, the protein expression may be detected by immunohistochemistry on tissue section of the tumor sample (e.g. frozen or formalin-fixed paraffin embedded material). The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with an antibody against the protein to be tested. A biological sample containing or suspected of containing the marker protein is then added to the coated wells. After a period of incubation sufficient to allow the formation of antibody-antigen complexes, the plate(s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

In a preferred embodiment, the determination of expression is determined by immunocytochemistry on cell preparations or immunohistochemistry on tissue sections of the tumor sample. Immunohistochemistry is not expansive, can be performed on routine formalin-fixed and paraffin-embedded tissue fragments, using documented and commercially available antibodies, and require only a small amount of tissue (4 $\mu$m per section and antibody per protein, i.e. per marker tested) that is easily obtained from paraffin blocks used for diagnosis. The reactions generally include revealing labels such as fluorescent, chemiluminescent, radioactive, enzymatic labels or dye molecules, or other methods for detecting the formation of a complex between the antigen and the antibody or antibodies reacted therewith. In a particular embodiment, the immunocytochemistry or immunohistochemistry is quantitative, so that expression level is determined.

For that purpose, it can be provided a diagnostic kit comprising antibodies directed against the proteins whose profiling is searched.

In a preferred embodiment, the expression level is determined by densitometry measurements, e.g. as described in the below examples.

In another embodiment, the expression may be determined by analyzing the presence of mRNA, or determining the quantity of mRNA.

Methods for detecting or determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous. Other methods of Amplification include ligase chain reaction (LCR), transcription-mediated amplification, strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

In another embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art.

In a particular embodiment, the expression level is determined by determining the number of copies of the genes. Comparative genomic hybridization (CGH) was developed to survey DNA copy-number variations across a whole genome. With CGH, differentially labelled test and reference genomic DNAs are co-hybridized to normal metaphase chromosomes, and fluorescence ratios along the length of chromosomes provide a cytogenetic representation of DNA copy-number variation. Array-based CGH, in which fluorescence ratios at arrayed DNA elements provide a locus-by-locus measure of DNA copy-number variation, represents another means of achieving increased mapping resolution. A cDNA microarray-based CGH method is described e.g. in Pollack et al, 1999.

*Determination of risk profile*

The method of the invention generally comprises determining the expression level of the marker proteins. The method of the invention still preferably comprises the step of comparing the combined expression level of said proteins with reference values, preferably by using computer tools.

Said "expression level of proteins" corresponds to the combined expression profile of said proteins, in the targeted subject or population. In the context of determining the quantity of protein, the "reference value" may be the mean of expression level determined in a whole cohort of patients.

Generally, the expression level as determined is a relative expression level which is correlated to a level of risk.

In a preferred embodiment, the expression level of the genes of interest is combined and processed according the known mathematical techniques single numerical "risk indices" or "risk scores" encompassing information from multiple cancer associated gene(s) inputs. Any suitable mathematical techniques may be used to derive appropriate classification functions from reference data. Such techniques may include discriminant function analysis, logistic regression, multiple regression, or other suitable statistical analysis techniques. Preferably, the invention provides a "risk score" according to a multivariate fractional polynomial logistic regression, as discussed for instance in reference (44, 45, 46, 47). This logistic regression can be readily provided by available softwares, such as NCSS and Statistica statistical software.

The relationship between the expression level of groups of predictive genes and the "risk score" can be evaluated without any cut-point but keeping marker values as continuous variables. To make it easier to read, the risk score can be correlated to a probability for the patient to develop metastasis. The risk score is then transformed:

$$\text{Probability of poor outcome} = \exp(-\text{risk score}) / [1 + \exp(-\text{risk score})]$$

The higher the probability is, the higher is the risk the patient shows a poor outcome or develops metastasis, classifying the patient as a candidate for chemotherapy.

In a most preferred embodiment, a sample, such as a tumor biopsy, is obtained from a patient to test. The expression of the markers is evaluated using immunohistochemistry and densitometry. The densitometry values are then subjected to a multivariate fractional polynomial logistic regression, which provides the following score, when the selected markers are p4E-BP1, HIF-1$\alpha$, Moesin, P21, P27, pAKT, PI3K, phospho MAPKAPK-2, pmTOR, SHARP-2:

$$\underline{\text{Logit}} : -.62602 + 3.2469\text{E-}02 \times \textbf{p4E-BP1} + .19086 \times \textbf{HIF-1}\alpha + 3.9232\text{E-}02 \times$$
$$\textbf{Moesin} + 2.9267\text{E-}02 \times \textbf{P21} + 7.1781\text{E-}02 \times \textbf{P27} + 8.30815\text{E-}02 \times \textbf{pAKT} +$$
$$7.7037\text{E-}02 \times \textbf{PI3K} + 7.4231\text{E-}02 \times \textbf{phospho MAPKAPK-2} + .31909 \times \textbf{pmTOR}$$
$$+ 4.973\text{E-}02 \times \textbf{SHARP-2}$$

(in bold: the densitometry value for each marker expression)

The Examples and figures illustrate the invention without limiting its scope.

## EXAMPLES

### Example 1: 10-marker signature of early distant metastasis risk in node-negative breast carcinomas

**MATERIALS AND METHODS**

*Patients*

The subjects were a consecutive series of 667 patients with invasive breast carcinomas who were operated on from 1996 to 2002 in the same department at the Hôpital Conception, Marseille. Surgery was in all cases the first treatment (PB). For this first step of treatment, patient management was handled by the same group of surgeons, and the oncologist's diagnosis was assessed by three senior pathologists. Conservative treatment and node resection (complete or sentinel) were applied according to current European recommendations. Likewise, radiotherapy, chemotherapy and hormone therapy were applied according to criteria currently used at that time.

Due to technical difficulties in performing immunocytochemical tests on many serial paraffin sections of a tissue microarray (TMA) to evaluate 64 different markers (see Table 1 B), complete data for all markers were finally obtained for only 572 patients out of the initial series of 667.

The 2007 follow-up data in clinical records (mean follow-up 102 months from 1996 to 2007) showed that 111/572 patients had distant metastases. Patients' age at diagnosis ranged from 40 to 65 years (mean 57 years). Tumors were pT1 b (32 %) and pT1 c (68 %). All were invasive ductal carcinomas, not otherwise specified and 29% were grade 1, 59 % grade 2 and 12 % grade 3 (Ellis and Elston grading method).

The present study focused mainly on correlation of quantitative immunohistochemical data with patients' outcome, independently of pT and tumor grade. Therefore current histoprognostic H & E staining data were not retained for statistical analysis, mainly to limit the burden of data and also to focus the statistical analysis on continuous variables homogeneously obtained by (numerical) densitometric measurements of immunoprecipitates obtained with the image analyzer.

*Tissues*

Tissue samples were all taken from surgical specimens after formalin fixation. Attention was paid to optimal and consistent tissue-handling procedures, including fast immersion in buffered formalin in an appropriate container by pathologists or by nurses trained in the procedure. Tumor samples were large and thick enough to allow subsequent TMA construction. Duration of fixation was 24 h for smaller samples (<5 cm) and 48 h for larger ones, to improve formalin penetration, before specimen dissection at room temperature. After fixation, paraffin pre-embedding and embedding were performed with currently available automated devices of the same brand.

All paraffin blocks were stored in the same room, where the temperature was maintained at 20°C prior to TMA construction.

*TMA construction*

The procedure for construction of TMAs was as previously described (22, 27, 40, 42, 43). Briefly, cores were punched from the selected 667 paraffin blocks, and distributed in three new blocks including two cores of 0.6 mm diameter for each tumor (about 220 cases per block). All the new blocks (TMAs) were stored at 4°C, and sections 4 $\mu$m thick were prepared for each marker to be examined by immunohistochemistry, using an automated microtome.

*Immunohistochemistry*

Serial tissue sections were prepared 24 h before immunohistochemical processing and stored at 4°C, as previously reported (22-28, 42-43). The immunoperoxidase procedure was performed using an automated Ventana Benchmark XT device that allowed similar well controlled antigen retrieval for all tumors, and Ventana kits.

Markers were detected using 64 commercially documented antibodies (Table 1B). Dilutions of each antibody were determined by pre-screening on the usual full 4 $\mu$m thick sections before use on TMA sections.

*Image analysis*

Densitometric measurements of immunoprecipitates in cores were assessed for each marker antibody in each individually identified core after digitization and image cropping of the slides, as previously reported (27, 28, 40) with a SAMBA 2050 automated device (SAMBA/TRIBVN Technologies, France).

*Statistical analysis*

Immunohistochemical expression of each marker was first correlated with patients' disease-free survival using NCSS and Statistica statistical software.

NCSS : 329 north mil East Kaysville UTAH 84033 USA

http://www.ncss.com

Statistica : StatSoft Inc, 2300 East 14th Street, Tulsa, OK 74104 USA

www.statsoft.com

When significant differences in mean quantitative scores of expression were identified between patients with or without distant metastasis or death (Mann-Whitney tests), the prognosis relevance was re evaluated for each marker using log rank test (Kaplan-Meier curves). the distribution and relationship of prognostic markers were documented through supervised hierarchical clusters and dendrograms, as previously reported (22, 28, 40, 42, 43, 45).

The next important step was to use logistic regression (and ROC curves) to identify the combination of markers (signature) with the best sensitivity and specificity for prediction of prognosis; the method of multivariable fractional polynomials was used to correlate the outcome variable with the quantitative immunohistochemical expression of markers as continuous variables without predetermined cut-points (44, 46, 47).

## RESULTS

### *Screening of potential markers of prognosis*

TMAs containing tumor samples from patients with (n = 111) or without distant metastasis (n = 461) were screened for immunoexpression of markers. Complete data for all 64 markers were available for 572 tumors (loss of some cores in TMAs occurred for 81 tumors). Depending on the marker evaluated, the immunostaining was observed within the cell nucleus, cytoplasm and/or plasma membrane.

The prognostic significance of markers was further individually evaluated by a univariate log rank test (Fig. 1) as previously described (22-28, 41, 42, 51, 52) and hierarchical clustering.

### *Logistic regression (ROC curves)*

The relationship between groups of predictive markers and the outcome variable was then evaluated by the multivariable fractional polynomials method (44, 46, 47), without any cut-point but keeping marker values as continuous variables.

The optimal combination found by the software from the image analysis data bank for the 64 markers in 572 node-negative breast carcinomas is shown in Tables 2 and 3 and Fig. 4. A ten-marker signature comprising PI3K, pmTOR, pMAP-K, SHARP-2, P21, HIF-1$\alpha$, moesin, p4EBP-1, p AKT and P27 was found to well classify 91.4 % of the patients in the category of either unfavorable or favorable prognosis with 93.7 % sensitivity (well classified 104/111) and 90.9 % specificity (well classified 419/461).

When the logistic regression was reassessed (Table 2) with exclusion of ER, PR and HER-2 (61 markers instead of 64), the same signature correctly classified patients in 91.61 % of the cases, suggesting that the above set of 10 markers constitutes a significant indicator of prognosis independently of hormone receptor and HER-2 amplification status.

When the multivariable fractional polynomials method was used on this series of 572 tumors, the estimated logistic regression model for adverse outcome (logit) for the 10-marker signature (quantitative score of 64 markers) was:

$$\underline{\text{Logit}} : -.62602 + 3.2469\text{E-}02 \times \textbf{p4E-BP1} + .19086\text{x } \textbf{HIF-1}\alpha + 3.9232\text{E-}02 \times$$

$$\textbf{Moesin} + 2.9267\text{E-}02 \times \textbf{P21} + 7.1781\text{E-}02 \times \textbf{P27} + 8.30815\text{E-}02 \times \textbf{pAKT} +$$

$$7.7037\text{E-}02 \times \textbf{PI3K} + 7.4231\text{E-}02 \times \textbf{phospho MAPKAPK-2} + .31909 \times \textbf{pmTOR}$$

$$+ 4.973\text{E-}02 \times \textbf{SHARP-2}$$

This model estimates "B" for a specific group (in the present analysis the group of patients with metastases), where logit (Y) = XB. To calculate the probability (x = densitometry / quick score for each marker) of classifying in the right category of outcome, the logit is transformed using Prob = exp (-logit) / [1 + exp (-logit)] or Prob = exp (- XB) / [1 + exp (-XB)]

Likewise for the 10 marker signature (PI3K, pmTOR, pMAP-K, SHARP-2, P21, HIF-1$\alpha$, moesin, p4EBP-1,

pAKT and P27) (quantitative scores of 61 markers, that is all 64 less ER, PR and c erb B2):

$$\underline{\text{Logit}} : -.50965 + 3.2708\text{E}{-02} \times \textbf{p4E-BP1} + .18267 \times \textbf{HIF-1}\alpha + 3.7484\text{E}{-02} \times$$
$$\textbf{Moesin} + 4.1069\text{E}{-02} \times \textbf{P21} + 8.4261\text{E}{-02} \times \textbf{pAKT} + 7.8838\text{E}{-02} \times \textbf{PI3K} +$$
$$7.0824\text{E}{-02} \times \textbf{phospho MAPKAPK-2} + .3126 \times \textbf{pmTOR} + 4.4381\text{E}{-02} \times$$
$$\textbf{SHARP2} + 5.2845\text{E}{-02} \times \textbf{P27}$$

and the probability was computed by the same procedure as indicated above.

**DISCUSSION**

The present assays contrast with the prior art assays which (i) usually require frozen tissue, that is not always easy to obtain or handle in routine clinical practice (or tissue briefly fixed in specific fixatives that are most often not appropriate for microscopic diagnosis), (ii) require a large amount of tissue although in small tumors detected at an early stage, the sampling can be detrimental to pathological diagnosis, and (iii) have very high cost, that is unlikely to be supportable by either an individual patient or public health insurance. Also, it has been shown that the large amount of data provided by gene expression microarrays is somewhat liable to misinterpretation (14-16).

In the present study, the inventors used a high-throughput quantitative immunohistochemical procedure as previously reported (42, 43) to analyse samples from a large series of patients with node-negative breast carcinoma (n = 572), in order to identify an optimal combination of markers allowing selection of patients who would not benefit from adjuvant therapy.

No previous study has been reported that used this approach of quantitative immunocytochemical tumor profiling for prediction of prognosis in node-negative breast carcinoma cases, although some quantitative immunohistochemical assays have been reported for a few individual markers (17-21).

Choosing cut-off points for positive staining of immunocytochemical prognostic markers in semi-quantitative analyses is a major issue that must be addressed before attempting statistical analysis and correlation with patients' outcome in order for this methodology to be usable in routine clinical practice. Methods relying upon dichotomizing continuous predictors using cut-points in multiple regression are controversial (14, 15, 44). In contrast, high-throughput quantification of immunoprecipitates by densitometry, using dedicated computerized devices properly and uniformly calibrated for immunohistochemical detection with a large tumor series in TMAs, provides an excellent method for comparison of subsets of tumors in a given cohort. It yields measurements appropriate for statistical analysis using continuous variables. Cut-point values of immunostaining can be determined according to log rank tests for determination of prognostic values by splitting patients in a series into two categories, those with tumors expressing the markers above or below the threshold predictive of disease-free survival. The cut-point definition can be then validated after stabilization on log rank p value curves before clinical use.

Importantly, in the present study the statistical approach was modified according to literature recommendations (44, 46, 47) by keeping continuous the values of densitometric measurements of the expression of potential predictors of prognosis. The node-negative tumors were quantitatively evaluated in TMA tissue sections by image analysis, using arbitrary units for densitometric measurements that are suitable for comparative studies of marker expression in variable clinical settings, particularly to correlate properly the expression of markers with the outcome variables. In this regard, using a multivariable fractional polynomials (44, 46, 47) method, the inventors analysed the correlation between marker expression and patient outcome without dichotomizing our tumor series into subsets by the use of a cut-point. All the 64 markers (and subsets of markers) were concomitantly analyzed according a non-linear model, in order to obtain the best combination of a limited number of markers with optimal accuracy, sensitivity and specificity. By this means, the inventors showed that a ten-marker signature (PI3K, pmTOR, pMAP-K, SHARP-2, P21, HIF-1$\alpha$, moesin, p4EBP-1, pAKT and P27) was sufficient to classify patients correctly in 91.43 % of the cases, independently of ER, PR and HER-2 status.

In conclusion, using a high-throughput procedure to profile 572 node-negative tumors in TMAs, computer-assisted densitometric quantification, logistic regression using continuous values of quantitative variables and a multivariable fractional polynomials approach to search for optimal combinations of a limited number of markers without any categorization through predefined cut-points, the inventors found a ten-marker signature that allowed correct classification of 91.61 % of the patients, independently of hormone receptor status, and that particularly identifies patients with low risk of early metastasis, who consequently should not require adjuvant chemotherapy.

**Table 2**

| | All markers (n = 64) | | | All markers less ER, RP, cerbB2 (n = 61) | |
|---|---|---|---|---|---|
| | Markers ranking | p deviance | | Markers ranking | p deviance |
| 1 | PI3K | < 0.0001 | 1 | PI3K | < 0.0001 |
| 2 | pmTOR | 0.0012 | 2 | pMAP-K | 0.0012 |
| 3 | pMAP-K | 0.0023 | 3 | pmTOR | 0.0021 |
| 4 | SHARP-2 | 0.0114 | 4 | HIF | 0.0065 |
| 5 | P21 | 0.0125 | 5 | Moesin | 0.01031 |
| 6 | HIF | 0.0133 | 6 | SHARP-2 | 0.0107 |
| 7 | Moesin | 0.0133 | 7 | P21 | 0.01463 |
| 8 | p4EBP-1 | 0.0292 | 8 | 4EBP-1 | 0.0363 |
| 9 | pAKT | 0.0366 | 9 | pAKT | 0.043 |
| 10 | P27 | 0.0399 | 10 | p27 | 0.049 |
| | 91.43% | | | 91.61 % | |

**Table 3: The combination of (10/64) markers relevance for patients selection for more aggressive therapy**

| All markers (cf Table 1) (n = 64) | Total patients | Well classified patients | Misclassified patients | Probability % |
|---|---|---|---|---|
| No metastasis | 461 | 419 | 42 | 90.86 |
| With metastasis | 111 | 104 | 7 | 93.69 |
| Total | 572 | 513 | 49 | 91.43 |
| All markers less ER, PR, HER-2 (n = 61) | | | | |
| No metastasis | 461 | 421 | 40 | 91.32 |
| With metastasis | 111 | 103 | 8 | 92.79 |
| Total | 572 | 524 | 48 | 91.61 |

**Example 2: Alternative marker signature of early distant metastasis risk in node-negative breast carcinomas**

The same method was used to test for the power of a signature including PI3K, Moesin, P21, pAKT, pMAP-K, pmTOR, p4E-BP-1, P27, PTEN, Gata3 and VEGF, without HIF-1$\alpha$.

This combination of markers allowed correct classification of 90.56% of the patients with node-negative breast carcinomas, with an area under the ROC curve of 0.954. The p values are:
PI3K (p<0.0001), Moesin (p=0.0073), P21 (p=0.00613), pAKT (p=0.0434), pMAP-K (p=0.0814), pmTOR (p=0.0003), p4E-BP-1 (p=0.0343), P27 (p=0.0247), PTEN (p=0.0264), Gata3 (p=0.0461) and VEGF (p=0.0475).

**Example 3: Marker signature of early distant metastasis risk in breast carcinomas**

The inventors have further shown that the markers have predictive value independently of the conditions of the ganglions (i.e. with N+ or without N- metastatic nodes).

The inventors have performed the same method of evaluation, as used in Examples 1 and 2, but this time with 905 patients (N+ and N-).

By this means, the inventors showed that a ten-marker signature (PI3K, STAT-1, P21, VEGF, pMAP-K, moesin, pAKT, caveolin, p4EBP-1, and pmTOR,) was sufficient to classify patients correctly in 88.84 % of the cases, independently of ER, PR and HER-2 status.

The sensitivity was 89.27% and the specificity was 87.38% (AUC of ROC curve = 0.929).

The p values are:

PI3K (p<0.0001), STAT-1 (p<0.0001), P21 (p=0.00041), VEGF (p=0.00194), pMAP-K (P=0.00227), moesin (p=0.00564), pAKT (p=0.0113), caveolin (p=0.019), p4EBP-1 (p=0.026), and pmTOR (p=0.037).

**REFERENCES**

1. Early Breast Cancer Trialists' Collaborative Group (EBCTCG). Effects of chemotherapy and hormonal therapy for early breast cancer on recurrence and 15-year survival: an overview of the randomised trials. Lancet 365: 1687-1717, 2005.

2. Goldhirsch A, Glick JH, Gelber RD, et al: Meeting highlights: international expert consensus on the primary therapy of early breast cancer 2005. Ann Oncol 16:1569-1583, 2005.

3. NCCN Clinical Practice Guidelines in Oncology. www.nccn.org, 2007.

4. Perou CM, Sorlie T, Eisen MB, et al: Molecular portraits of human breast tumors. Nature 406:747-752, 2000.

5. van de Vijver MJ, He YD, van't Veer LJ, et al: A gene-expression signature as a predictor of survival in breast cancer. N Engl J Med 347:1999-2009, 2002.

6. van 't Veer LJ, Dai H, van de Vijver MJ, et al: Gene expression profiling predicts clinical outcome of breast cancer. Nature 415:530-536, 2002.

7. Sorlie T, Tibshirani R, Parker J, et al: Repeated observation of breast tumor subtypes in independent gene expression data sets. Proc Nat Acad Sci U S A 100:8418-8423, 2003.

8. Bertucci F, Finetti P, Rougemont J, et al: Gene expression profiling for molecular characterization of inflammatory breast cancer and prediction of response to chemotherapy. Cancer Res 64:8558-8565, 2004.

9. Pittman J, Huang E, Dressman H, et al: Integrated modeling of clinical and gene expression information for personalized prediction of disease outcomes. Proc Natl Acad Sci U S A 101:8431-8436, 2004.

10. Paik S, Shak S, Tang G, et al: A multigene assay to predict recurrence of tamoxifen-treated, node-negative breast cancer. N Engl J Med 351:2817-2826, 2004.

11. Chang HY, Nuyten DS, Sneddon JB, et al: Robustness, scalability, and integration of a wound-response gene expression signature in predicting breast cancer survival. Proc Natl Acad Sci U S A 102:3738-3743, 2005.

12. Buyse M, Loi S, van't Veer L, et al: Validation and clinical utility of a 70-gene prognostic signature for women with node-negative breast cancer. J Natl Cancer Inst 98:1183-1192, 2006.

13. Wang Y, Klijn JG, Zhang Y, et al. Gene-expression profiles to predict distant metastasis of lymph-node-negative primary breast cancer. Lancet 365:671-679, 2005.

14. Dunkler D, Michiels S, Schemper M: Gene expression profiling: does it add predictive accuracy to clinical characteristics in cancer prognosis? Eur J Cancer 43:745-751, 2007.

15. Michiels S, Koscielny S, Hill C: Prediction of cancer outcome with microarrays: a multiple random validation strategy. Lancet 365:488-492, 2005.

16. Simon R, Radmacher MD, Dobbin K, et al: Pitfalls in the use of DNA microarray data for diagnostic and prognostic classification. J Natl Cancer Inst 95:14-18, 2003.

17. Camp RL, Chung GG, Rimm DL: Automated subcellular localization and quantification of protein expression in tissue microarrays. Nat Med 8:1323-1327, 2002.

18. Camp RL, Rimm EB, Rimm DL: Met expression is associated with poor outcome in patients with axillary lymph node negative breast carcinoma. Cancer 86:2259-2265, 1999.

19. Chung GG, Zerkowski MP, Ghosh S, et al: Quantitative analysis of estrogen receptor heterogeneity in breast cancer. Lab Invest 87:662-669, 2007.

20. Moeder CB, Giltnane JM, Harigopal M, et al: Quantitative justification of the change from 10 % to 30 % for human epidermal growth factor receptor 2 scoring in the American Society of Clinical Oncology/College of American Pathologists guidelines: Tumor heterogeneity in breast cancer and its implications for tissue microarray based assessment of outcome. J Clin Oncol 25:5418-5425, 2007.

21. Pozner-Moulis S, Cregger M, Camp RL, et al: Antibody validation by quantitative analysis of protein expression using expression of Met in breast cancer as a model. Lab Invest 87:251-260, 2007.

22. Charpin C, Dales JP, Garcia S, et al: Tumor neoangiogenesis by CD31 and CD105 expression evaluation in breast carcinoma tissue microarrays. Clin Cancer Res 10:5815-5819,2004.

23. Charpin C, Garcia S, Bonnier P, et al: bcl-2 automated and quantitative immunocytochemical assays in breast carcinomas:correlation with 10-year follow-up. J Clin Oncol 16:2025-2031, 1998.

24. Charpin C, Garcia S, Bouvier C, et al: Automated and quantitative immunocytochemical assays of CD44v6 in breast carcinomas. Hum Pathol 28:289-296, 1997.

25. Charpin C, Garcia S, Bouvier C, et al: CD31/PECAM automated and quantitative immunocytochemical assays in breast carcinomas:correlation with patient follow-up. Am J Clin Pathol 107:534-541, 1997.

26. Charpin C, Vielh P, Duffaud F, et al: Quantitative immunocytochemical assays of P-glycoprotein in breast carcinomas:correlation to messenger RNA expression and to immunohistochemical prognostic indicators. J Natl Cancer Inst 86:1539-1545, 1994.

27. Garcia S, Dales JP, Charafe-Jauffret E, et al: Overexpression of c-Met and of the transducers PI3K, FAK and JAK in breast carcinomas correlates with shorter survival and neoangiogenesis. Int J Oncol 31:49-58, 2007.

28. Garcia S, Dales JP, Jacquemier J, et al: c-Met overexpression in inflammatory breast carcinomas:automated quantification on tissue microarrays. Br J Cancer 96:329-335, 2007.

29. Makretsov NA, Huntsman DG, Nielsen TO, et al: Hierarchical clustering analysis of tissue microarray immunostaining data identifies prognostically significant groups of breast carcinoma. Clin Cancer Res 10:6143-6151, 2004.

30. Nielsen TO, Hsu FD, Jensen K, et al: Immunohistochemical and clinical characterization of the basal-like subtype of invasive breast carcinoma. Clin Cancer Res 10:5367-5374, 2004.

31. Ginestier C, Charafe-Jauffret E, Bertucci F, et al: Distinct and complementary information provided by use of tissue and DNA microarrays in the study of breast tumor markers. Am J Pathol 161:1223-1233, 2002.

32. Jacquemier J, Ginestier C, Rougemont J, et al: Protein expression profiling identifies subclasses of breast cancer and predicts prognosis. Cancer Res 65:767-779, 2005.

33. Charafe-Jauffret E, Ginestier C, Monville F, et al: Gene expression profiling of breast cell lines identifies potential new basal markers. Oncogene 25:2273-2284, 2006.

34. Livasy CA, Karaca G, Nanda R, et al: Phenotypic evaluation of the basal-like subtype of invasive breast carcinoma. Mod Pathol 19:264-271, 2006.

35. Charafe-Jauffret E, Monville F, Bertucci F, et al: Moesin expression is a marker of basal breast carcinomas. Int J Cancer 121:1779-1785, 2007.

36. Laakso M, Tanner M, Nilsson J, et al: Basoluminal carcinoma:a new biologically and prognostically distinct entity between basal and luminal breast cancer. Clin Cancer Res 12:4185-4191, 2006.

37. Da Silva L, Clarke C, Lakhani SR. Demystifying basal-like breast carcinomas. J Clin Pathol 60:1328-1332, 2007.

38. Reis-Filho JS, Tutt AN. Triple negative tumors: a critical review. Histopathology 52:108-118, 2008.

39. Rakha EA, El-Sayed ME, Green AR, et al: Breast carcinoma with basal differentiation: a proposal for pathology definition based on basal cytokeratin expression. Histopathology 50:434-438, 2007.

40. Garcia S, Dales JP, Charafe-Jauffret E, et al: Poor prognosis in breast carcinomas correlates with increased expression of targetable CD146 and c-Met and with proteomic basal-like phenotype. Hum Pathol 38:830-841, 2007.

41. Jacquemier J, Padovani L, Rabayrol L, et al: Typical medullary breast carcinomas have a basal/myoepithelial phenotype. J Pathol 207:260-268, 2005.

42. Charpin C, Giusiano S, Secq V, et al: A signature predictive of disease outcome in breast carcinomas, identified by quantitative immunocytochemical assays. Int J Cancer, 124:2124-2134, 2009.

43. Charpin C, Giusiano S, Secq V, et al: Quantitative immunocytochemical profiling predictive of early outcome of disease in triple-negative breast carcinomas. Int J Oncology, 34:983-993, 2009.

44. Royston P, Douglas G, Sauerbrei A, et al: Dichotomizing continuous predictors in multiple regression: a bad idea. Stat Med, 25:127-141, 2006.

45. Altman DG, Lausen B, Sauerbrei W, et al: Dangers of using "optimal" cutpoints in the evaluation of prognostic factors. J Natl Cancer Inst 86:829-835, 1994.

46. Von Elm E, Altman DG, Egger M, et al: The strengthening the reporting of observational studies in epidemiology (STROBE) statement: Guidelines for reporting observational studies. Lancet, 370 :1453-1457, 2007

47. Von Elm E, Altman DG, Egger M, et al: The strengthening the reporting of observational studies in epidemiology (STROBE) statement: Guidelines for reporting observational studies. J Clin Epidem., 61:344-349, 2008.

48. Darb-Esfahani S, Faggad A, Noske, et al: Phospho-mTOR and phospho-4EBP1 in endometrial adenocarcinoma : association with stage and grade in vivo and link with response to rapamycin treatment in vitro. J Cancer Res Clin Oncol, 2008.

49. Rao RD, Buckner JC, Sarkaria JN:Mammalian target of rapamycin (mTOR) inhibitors as anti-cancer agents. Curr Cancer Drug Targets. 4:621-635, 2004.

50. Boulay A, Rudloff J, Ye Y, et al: Dual inhibition of mTOR and estrogen receptor signaling in vitro induces cell death in models of breast cancer. Clin Cancer Res, 11:5319-5328, 2005.

51. Pouyssegur J, Dayan F, Mazure NM: Hypoxia signalling in cancer and approaches to enforce tumour regression. Nature, 441 :437-443, 2006.

52. Dales JP, Garcia S, Meunier-Carpentier S, et al: Overexpression of hypoxia-inducible factor HIF-1alpha predicts early relapse in breast cancer: retrospective study in a series of 745 patients. Int J Cancer 116:734-739, 2005.

53. Gort EH, Groot AJ, Derks van de Ven TLP, et al: Hypoxia-inducible factor-1a expression requires PI 3-kinase activity and correlates with Akt1 phosphorylation in invasive breast carcinomas. Oncogene 1-5, 2006

54. Helczynska K, Larsson A-M, Holmquist Mengelbier L, et al: Hypoxia-inducible factor-2a correlates to distant recurrence and poor outcome in invasive breast cancer. Cancer Res 68:9212-9220, 2008.

55. Dales JP, Garcia S, Carpentier S, et al: Prediction of metastasis risk (11 year follow-up) using VEGF-R1, VEGF-R2, Tie-2/Tek and CD105 expression in breast cancer (n=905). Br J Cancer 90:1216-1221, 2004.

**Claims**

1. An *in vitro* method for predicting clinical outcome of a patient affected with a breast carcinoma, which method comprises determining the expression of at least two proteins in a biological sample of said patient, wherein one of them is PI3K and one other is Moesin, pAKT, or pMAP-K.

2. The method of claim 1, wherein the patient is affected with an node-negative breast carcinoma.

3. The method of any of claims 1 or 2, wherein expression of said proteins is indicative of a patient with poor clinical outcome or who would benefit from a chemotherapeutic treatment.

4. The method of any of claims 1 to 3, further comprising the step of determining the expression levels of said proteins and comparing the combined expression level of said proteins with reference values.

5. The method of any of claims 1 to 4, comprising quantifying the expression level of said proteins and subjecting the obtained values to a logistic regression, such as a multivariate fractional polynomial logistic regression, whereby a risk score is obtained which correlates to the probability of the patient to show a poor clinical outcome.

6. The method of claim 5, wherein the probability is calculated as:

$$\text{Probability of poor outcome} = \exp(-\text{risk score}) / [1 + \exp(-\text{risk score})]$$

7. The method of any of claims 1 to 6, wherein the biological sample is a breast tumor tissue biopsy.

8. The method of any of claims 1 to 7, wherein the expression is determined by quantifying the proteins.

9. The method of any of claims 1 to 8, wherein the expression is determined by immunohistochemistry.

10. The method of any of claims 1 to 7, wherein the expression is determined by quantifying the level of mRNA coding for said proteins.

11. The method of any of claims 1 to 10, comprising determining the expression of the proteins selected from the groups consisting of:

    a. PI3K and Moesin
    b. PI3K and pAKT and pMAP-K and
    c. PI3K and Moesin and pMAP-K

12. The method of any of claims 1 to 11, further comprising determining the expression of P21.

13. The method of claim 12, comprising determining the expression of PI3K, Moesin, P21 and pAKT.

14. The method of claim 13, further comprising determining the expression of SHARP2 and/or pMAP-K.

15. The method of claim 13 or 14, further comprising determining the expression of pmTOR and/or VEGF.

16. The method of claim 14, comprising determining the expression of PI3K, Moesin, P21, pAKT, SHARP2, pMAP-K, and optionally HIF-1$\alpha$.

17. The method of claim 14, comprising determining the expression of PI3K, Moesin, P21, pAKT, pMAP-K, VEGF, and optionally pmTOR.

18. The method of claim 14, comprising determining the expression of PI3K, Moesin, P21, pAKT, SHARP2, pMAP-K, pmTOR, p4E-BP-1, P27, and HIF-1$\alpha$.

19. The method of claim 14, comprising determining the expression of PI3K, Moesin, P21, pAKT, pMAP-K, pmTOR, p4E-BP-1, P27, PTEN, Gata3 and VEGF.

20. The method of claim 14, comprising determining the expression of PI3K, STAT-1, P21, VEGF, pMAP-K, moesin, pAKT, caveolin, p4EBP-1, and pmTOR.

21. The method of any of claims 1 to 20, comprising determining the expression of at least 30 genes, preferably at least 40 genes, preferably at least 50 genes, selected from the group of genes as shown in Table 1 B.

FIGURE 1

Well classified : 91.2 %
Area under ROC curve : 0.95

Well classified : 91.6 %
Area under ROC curve : 0.96

Well classified : 90 %
Area under ROC curve : 0.94

FIGURE 2

Europäisches
Patentamt

European
Patent Office

Office européen
des brevets

# EUROPEAN SEARCH REPORT

**Application Number**

EP 09 30 5701

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHARPIN COLETTE ET AL: "A signature predictive of disease outcome in breast carcinomas, identified by quantitative immunocytochemical assays" INTERNATIONAL JOURNAL OF CANCER, vol. 124, no. 9, May 2009 (2009-05), pages 2124-2134, XP002543639 ISSN: 0020-7136 | 1-12,21 | INV. C12Q1/698 |
| Y | * tables 1,2 * <br> * page 2127, column 1, lines 3-15 * <br> * page 2130, column 1, paragraph 24-29 * <br> * the whole document * | 13-20 | |
| Y | CHARPIN C ET AL: "Quantitative immunohistochemical expression of c Kit in breast carcinomas is predictive of patients' outcome" BRITISH JOURNAL OF CANCER, vol. 101, no. 1, June 2009 (2009-06), pages 48-54, XP002543640 ISSN: 0007-0920(print) 1532-1827(ele * tables 1-3 * <br> * the whole document * | 13-20 | |
| Y | GARCIA S ET AL: "c-Met overexpression in inflammatory breast carcinomas: automated quantification on tissue microarrays" BRITISH JOURNAL OF CANCER, vol. 96, no. 2, January 2007 (2007-01), pages 329-335, XP002543641 ISSN: 0007-0920(print) 1532-1827(ele * tables 1-3 * <br> * the whole document * | 13-20 | |

**TECHNICAL FIELDS SEARCHED (IPC)**

C12Q

-/--

The present search report has been drawn up for all claims

| Place of search | Date of completion of the search | Examiner |
|---|---|---|
| Munich | 24 September 2009 | Helliot, Bertrand |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................................

& : member of the same patent family, corresponding
document

EPO FORM 1503 03.82 (P04C01)

| | Europäisches Patentamt European Patent Office Office européen des brevets | **EUROPEAN SEARCH REPORT** | Application Number EP 09 30 5701 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GARCIA STEPHANE ET AL: "Overexpression of c-Met and of the transducers PI3K, FAK and JAK in breast carcinomas correlates with shorter survival and neoangiogenesis" INTERNATIONAL JOURNAL OF ONCOLOGY, vol. 31, no. 1, July 2007 (2007-07), pages 49-58, XP002543642 ISSN: 1019-6439 * tables 1-3 * * the whole document * ----- | 13-20 | |
| Y | GARCIA ET AL: "Poor prognosis in breast carcinomas correlates with increased expression of targetable CD146 and c-Met and with proteomic basal-like phenotype" HUMAN PATHOLOGY, SAUNDERS, PHILADELPHIA, PA, US, vol. 38, no. 6, 23 May 2007 (2007-05-23), pages 830-841, XP022089969 ISSN: 0046-8177 * tables 2-4 * * the whole document * ----- | 13-20 | |
| Y | MEUNIER-CARPENTIER S ET AL: "COMPARISON OF THE PROGNOSIS INDICATION OF VEGFR-1 AND VEGFR-2 AND TIE2 RECEPTOR EXPRESSION IN BREAST CARCINOMA" INTERNATIONAL JOURNAL OF ONCOLOGY, DEMETRIOS A. SPANDIDOS ED. & PUB, GR, vol. 26, no. 4, 1 January 2005 (2005-01-01), pages 977-984, XP009067882 ISSN: 1019-6439 * abstract * * the whole document * ----- | 13-20 | TECHNICAL FIELDS SEARCHED (IPC) |

-/--

The present search report has been drawn up for all claims

| Place of search | Date of completion of the search | Examiner |
|---|---|---|
| Munich | 24 September 2009 | Helliot, Bertrand |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 2 278 026 A1

# EUROPEAN SEARCH REPORT

**Application Number**

EP 09 30 5701

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHARPIN COLETTE ET AL: "Quantitative immunocytochemical profile to predict early outcome of disease in triple-negative breast carcinomas" INTERNATIONAL JOURNAL OF ONCOLOGY, DEMETRIOS A. SPANDIDOS ED. & PUB, GR, vol. 34, no. 4, 1 April 2009 (2009-04-01), pages 983-993, XP009122065 ISSN: 1019-6439 * tables 1-3 * * the whole document * | 13-20 | |
| Y | KIRKEGAARD TOVE ET AL: "AKT activation predicts outcome in breast cancer patients treated with tamoxifen" JOURNAL OF PATHOLOGY, vol. 207, no. 2, October 2005 (2005-10), pages 139-146, XP002543643 ISSN: 0022-3417 * tables 1,2 * * the whole document * | 13-20 | |
| Y | EP 1 918 386 A (GENOMIC HEALTH INC [US]) 7 May 2008 (2008-05-07) * paragraph [0104] * * the whole document * | 13-20 | TECHNICAL FIELDS SEARCHED (IPC) |
| Y | WO 2007/047754 A (LIOTTA LANCE A [US]; PETRICOIN EMANUEL F III [US]; ESPINA VIRGINIA [US] 26 April 2007 (2007-04-26) * page 14, lines 5-9 * * the whole document * | 13-20 | |

-/--

The present search report has been drawn up for all claims

| Place of search | Date of completion of the search | Examiner |
|---|---|---|
| Munich | 24 September 2009 | Helliot, Bertrand |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

23

**EUROPEAN SEARCH REPORT**

Application Number

EP 09 30 5701

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GORI S ET AL: "EGFR, pMAPK, pAkt and PTEN status by immunohistochemistry: Correlation with clinical outcome in HER2-positive metastatic breast cancer patients treated with trastuzumab" ANNALS OF ONCOLOGY 2009 GB, vol. 20, no. 4, 2009, pages 648-654, XP009122090 ISSN: 0923-7534 1569-8041 * abstract * * the whole document * ----- | 13-20 | |

TECHNICAL FIELDS SEARCHED (IPC)

The present search report has been drawn up for all claims

| Place of search | Date of completion of the search | Examiner |
|---|---|---|
| Munich | 24 September 2009 | Helliot, Bertrand |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 30 5701

This annex lists the patent family members relating to the patent documents cited in the above-mentioned European search report.
The members are as contained in the European Patent Office EDP file on
The European Patent Office is in no way liable for these particulars which are merely given for the purpose of information.

24-09-2009

| Patent document cited in search report | | Publication date | Patent family member(s) | | Publication date |
|---|---|---|---|---|---|
| EP 1918386 | A | 07-05-2008 | NONE | | |
| WO 2007047754 | A | 26-04-2007 | CA | 2626456 A1 | 26-04-2007 |
| | | | CN | 101421625 A | 29-04-2009 |
| | | | EP | 1946120 A2 | 23-07-2008 |
| | | | JP | 2009511935 T | 19-03-2009 |
| | | | US | 2009148859 A1 | 11-06-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Early Breast Cancer Trialists' Collaborative Group (EBCTCG). Effects of chemotherapy and hormonal therapy for early breast cancer on recurrence and 15-year survival: an overview of the randomised trials. *Lancet,* 2005, vol. 365, 1687-1717
- **Goldhirsch A ; Glick JH ; Gelber RD et al.** Meeting highlights: international expert consensus on the primary therapy of early breast cancer 2005. *Ann Oncol,* 2005, vol. 16, 1569-1583
- *NCCN Clinical Practice Guidelines in Oncology,* 2007, www.nccn.org
- **Perou CM ; Sorlie T ; Eisen MB et al.** Molecular portraits of human breast tumors. *Nature,* 2000, vol. 406, 747-752
- **van de Vijver MJ ; He YD ; van't Veer LJ et al.** A gene-expression signature as a predictor of survival in breast cancer. *N Engl J Med,* 2002, vol. 347, 1999-2009
- **van 't Veer LJ ; Dai H ; van de Vijver MJ et al.** Gene expression profiling predicts clinical outcome of breast cancer. *Nature,* 2002, vol. 415, 530-536
- **Sorlie T ; Tibshirani R ; Parker J et al.** Repeated observation of breast tumor subtypes in independent gene expression data sets. *Proc Nat Acad Sci U S A,* 2003, vol. 100, 8418-8423
- **Bertucci F ; Finetti P ; Rougemont J et al.** Gene expression profiling for molecular characterization of inflammatory breast cancer and prediction of response to chemotherapy. *Cancer Res,* 2004, vol. 64, 8558-8565
- **Pittman J ; Huang E ; Dressman H et al.** Integrated modeling of clinical and gene expression information for personalized prediction of disease outcomes. *Proc Natl Acad Sci U S A,* 2004, vol. 101, 8431-8436
- **Paik S ; Shak S ; Tang G et al.** A multigene assay to predict recurrence of tamoxifen-treated, node-negative breast cancer. *N Engl J Med,* 2004, vol. 351, 2817-2826
- **Chang HY ; Nuyten DS ; Sneddon JB et al.** Robustness, scalability, and integration of a wound-response gene expression signature in predicting breast cancer survival. *Proc Natl Acad Sci U S A,* 2005, vol. 102, 3738-3743
- **Buyse M ; Loi S ; van't Veer L et al.** Validation and clinical utility of a 70-gene prognostic signature for women with node-negative breast cancer. *J Natl Cancer Inst,* 2006, vol. 98, 1183-1192

- **Wang Y ; Klijn JG ; Zhang Y et al.** Gene-expression profiles to predict distant metastasis of lymph-node-negative primary breast cancer. *Lancet,* 2005, vol. 365, 671-679
- **Dunkler D ; Michiels S ; Schemper M.** Gene expression profiling: does it add predictive accuracy to clinical characteristics in cancer prognosis?. *Eur J Cancer,* vol. 43, 745-751
- **Michiels S ; Koscielny S ; Hill C.** Prediction of cancer outcome with microarrays: a multiple random validation strategy. *Lancet,* 2005, vol. 365, 488-492
- **Simon R ; Radmacher MD ; Dobbin K et al.** Pitfalls in the use of DNA microarray data for diagnostic and prognostic classification. *J Natl Cancer Inst,* 2003, vol. 95, 14-18
- **Camp RL ; Chung GG ; Rimm DL.** Automated subcellular localization and quantification of protein expression in tissue microarrays. *Nat Med,* 2002, vol. 8, 1323-1327
- **Camp RL ; Rimm EB ; Rimm DL.** Met expression is associated with poor outcome in patients with axillary lymph node negative breast carcinoma. *Cancer,* 1999, vol. 86, 2259-2265
- **Chung GG ; Zerkowski MP ; Ghosh S et al.** Quantitative analysis of estrogen receptor heterogeneity in breast cancer. *Lab Invest,* 2007, vol. 87, 662-669
- **Moeder CB ; Giltnane JM ; Harigopal M et al.** Quantitative justification of the change from 10 % to 30 % for human epidermal growth factor receptor 2 scoring in the American Society of Clinical Oncology/College of American Pathologists guidelines: Tumor heterogeneity in breast cancer and its implications for tissue microarray based assessment of outcome. *J Clin Oncol,* 2007, vol. 25, 5418-5425
- **Pozner-Moulis S ; Cregger M ; Camp RL et al.** Antibody validation by quantitative analysis of protein expression using expression of Met in breast cancer as a model. *Lab Invest,* 2007, vol. 87, 251-260
- **Charpin C ; Dales JP ; Garcia S et al.** Tumor neoangiogenesis by CD31 and CD105 expression evaluation in breast carcinoma tissue microarrays. *Clin Cancer Res,* 2004, vol. 10, 5815-5819
- **Charpin C ; Garcia S ; Bonnier P et al.** bcl-2 automated and quantitative immunocytochemical assays in breast carcinomas:correlation with 10-year follow-up. *J Clin Oncol,* 1998, vol. 16, 2025-2031

- **Charpin C ; Garcia S ; Bouvier C et al.** Automated and quantitative immunocytochemical assays of CD44v6 in breast carcinomas. *Hum Pathol,* 1997, vol. 28, 289-296
- **Charpin C ; Garcia S ; Bouvier C et al.** CD31/PECAM automated and quantitative immunocytochemical assays in breast carcinomas:correlation with patient follow-up. *Am J Clin Pathol,* 1997, vol. 107, 534-541
- **Charpin C ; Vielh P ; Duffaud F et al.** Quantitative immunocytochemical assays of P-glycoprotein in breast carcinomas:correlation to messenger RNA expression and to immunohistochemical prognostic indicators. *J Natl Cancer Inst,* 1994, vol. 86, 1539-1545
- **Garcia S ; Dales JP ; Charafe-Jauffret E et al.** Overexpression of c-Met and of the transducers PI3K, FAK and JAK in breast carcinomas correlates with shorter survival and neoangiogenesis. *Int J Oncol,* 2007, vol. 31, 49-58
- **Garcia S ; Dales JP ; Jacquemier J et al.** c-Met overexpression in inflammatory breast carcinomas:automated quantification on tissue microarrays. *Br J Cancer,* 2007, vol. 96, 329-335
- **Makretsov NA ; Huntsman DG ; Nielsen TO et al.** Hierarchical clustering analysis of tissue microarray immunostaining data identifies prognostically significant groups of breast carcinoma. *Clin Cancer Res,* 2004, vol. 10, 6143-6151
- **Nielsen TO ; Hsu FD ; Jensen K et al.** Immunohistochemical and clinical characterization of the basal-like subtype of invasive breast carcinoma. *Clin Cancer Res,* 2004, vol. 10, 5367-5374
- **Ginestier C ; Charafe-Jauffret E ; Bertucci F et al.** Distinct and complementary information provided by use of tissue and DNA microarrays in the study of breast tumor markers. *Am J Pathol,* 2002, vol. 161, 1223-1233
- **Jacquemier J ; Ginestier C ; Rougemont J et al.** Protein expression profiling identifies subclasses of breast cancer and predicts prognosis. *Cancer Res,* 2005, vol. 65, 767-779
- **Charafe-Jauffret E ; Ginestier C ; Monville F et al.** Gene expression profiling of breast cell lines identifies potential new basal markers. *Oncogene,* 2006, vol. 25, 2273-2284
- **Livasy CA ; Karaca G ; Nanda R et al.** Phenotypic evaluation of the basal-like subtype of invasive breast carcinoma. *Mod Pathol,* 2006, vol. 19, 264-271
- **Charafe-Jauffret E ; Monville F ; Bertucci F et al.** Moesin expression is a marker of basal breast carcinomas. *Int J Cancer,* 2007, vol. 121, 1779-1785
- **Laakso M ; Tanner M ; Nilsson J et al.** Basoluminal carcinoma:a new biologically and prognostically distinct entity between basal and luminal breast cancer. *Clin Cancer Res,* 2006, vol. 12, 4185-4191
- **Da Silva L ; Clarke C ; Lakhani SR.** Demystifying basal-like breast carcinomas. *J Clin Pathol,* 2007, vol. 60, 1328-1332
- **Reis-Filho JS ; Tutt AN.** Triple negative tumors: a critical review. *Histopathology,* 2008, vol. 52, 108-118
- **Rakha EA ; El-Sayed ME ; Green AR et al.** Breast carcinoma with basal differentiation: a proposal for pathology definition based on basal cytokeratin expression. *Histopathology,* 2007, vol. 50, 434-438
- **Garcia S ; Dales JP ; Charafe-Jauffret E et al.** Poor prognosis in breast carcinomas correlates with increased expression of targetable CD146 and c-Met and with proteomic basal-like phenotype. *Hum Pathol,* 2007, vol. 38, 830-841
- **Jacquemier J ; Padovani L ; Rabayrol L et al.** Typical medullary breast carcinomas have a basal/myoepithelial phenotype. *J Pathol,* 2005, vol. 207, 260-268
- **Charpin C ; Giusiano S ; Secq V et al.** A signature predictive of disease outcome in breast carcinomas, identified by quantitative immunocytochemical assays. *Int J Cancer,* 2009, vol. 124, 2124-2134
- **Charpin C ; Giusiano S ; Secq V et al.** Quantitative immunocytochemical profiling predictive of early outcome of disease in triple-negative breast carcinomas. *Int J Oncology,* 2009, vol. 34, 983-993
- **Royston P ; Douglas G ; Sauerbrei A et al.** Dichotomizing continuous predictors in multiple regression: a bad idea. *Stat Med,* 2006, vol. 25, 127-141
- **Altman DG ; Lausen B ; Sauerbrei W et al.** Dangers of using "optimal" cutpoints in the evaluation of prognostic factors. *J Natl Cancer Inst,* 1994, vol. 86, 829-835
- **Von Elm E ; Altman DG ; Egger M et al.** The strengthening the reporting of observational studies in epidemiology (STROBE) statement: Guidelines for reporting observational studies. *Lancet,* 2007, vol. 370, 1453-1457
- **Von Elm E ; Altman DG ; Egger M et al.** The strengthening the reporting of observational studies in epidemiology (STROBE) statement: Guidelines for reporting observational studies. *J Clin Epidem,* 2008, vol. 61, 344-349
- **Darb-Esfahani S ; Faggad A ; Noske et al.** Phospho-mTOR and phospho-4EBP1 in endometrial adenocarcinoma :association with stage and grade in vivo and link with response to rapamycin treatment in vitro. *J Cancer Res Clin Oncol,* 2008
- **Rao RD ; Buckner JC ; Sarkaria JN.** Mammalian target of rapamycin (mTOR) inhibitors as anti-cancer agents. *Curr Cancer Drug Targets.,* 2004, vol. 4, 621-635
- **Boulay A ; Rudloff J ; Ye Y et al.** Dual inhibition of mTOR and estrogen receptor signaling in vitro induces cell death in models of breast cancer. *Clin Cancer Res,* 2005, vol. 11, 5319-5328

- **Pouyssegur J ; Dayan F ; Mazure NM.** Hypoxia signalling in cancer and approaches to enforce tumour regression. *Nature,* 2006, vol. 441, 437-443
- **Dales JP ; Garcia S ; Meunier-Carpentier S et al.** Overexpression of hypoxia-inducible factor HIF-1alpha predicts early relapse in breast cancer: retrospective study in a series of 745 patients. *Int J Cancer,* 2005, vol. 116, 734-739
- **Gort EH ; Groot AJ ; Derks van de Ven TLP et al.** Hypoxia-inducible factor-1a expression requires PI 3-kinase activity and correlates with Akt1 phosphorylation in invasive breast carcinomas. *Oncogene,* 2006, 1-5

- **Helczynska K ; Larsson A-M ; Holmquist Mengelbier L et al.** Hypoxia-inducible factor-2a correlates to distant recurrence and poor outcome in invasive breast cancer. *Cancer Res,* 2008, vol. 68, 9212-9220
- **Dales JP ; Garcia S ; Carpentier S et al.** Prediction of metastasis risk (11 year follow-up) using VEGF-R1, VEGF-R2, Tie-2/Tek and CD105 expression in breast cancer (n=905). *Br J Cancer,* 2004, vol. 90, 1216-1221